# EUROPEAN PATENT SPECIFICATION

(11) **EP 2 803 656 B1**
(45) Date of publication and mention of the grant of the patent: **27.02.2019**
(21) Application number: 14168593.3
(22) Date of filing: 16.05.2014
(51) Int. Cl.: C07C 7/13

(54) **Method and device for separating a gas mixture comprising carbon dioxide and methane**
Vorrichtung und Verfahren zum Scheiden einer Kohlendioxid und Methan enthaltende Gasmischung
Procédé et dispositif pour séparer un mélange gazeux contenant du dioxide de carbone et du méthane

(30) Priority: 16.05.2013 NL 2010816
(43) Date of publication of application: 19.11.2014
(73) Proprietor: Green Vision Holding B.V., 6827 AV Arnhem (NL)
(72) Inventor: Westendorp, Gerard, 6671 BL Zetten (NL); Oudenhoven, Tom Martin Jacob, 6831 MH Arnhem (NL); Wang, Hugui, 6545 HS Nijmegen (NL); Der Kinderen, Joannes Maria, 7391 SJ Twello (NL); Rep, Marco, 6846 HN Arnhem (NL)
(74) Representative: Dokter, Hendrik Daniel

(56) References cited:
- EP-A1- 0 314 040
- EP-A2- 0 193 716
- WO-A2-2006/052937

## Description

The invention relates to a method for separating a first gas mixture comprising carbon dioxide (CO₂) and methane (CH₄) into a second gas mixture with a relatively high methane content and a third gas mixture with a relatively high carbon dioxide content, comprising the steps of (i) providing the first gas mixture, (ii) admitting the first gas mixture into at least one vessel in which an adsorption mass for adsorbing carbon dioxide is received, (iii) discharging the separated second gas mixture from the vessel, and (iv) discharging the third gas mixture from the vessel.

An example of a gas mixture comprising carbon dioxide and methane is biogas. Biogas is seen as an important potential renewable energy source. Biogas is for instance released at landfill sites. This gas can be extracted from the landfill site and can then be processed into a useful energy carrier.

This production of biogas from (bio)waste can be speeded up by utilizing a fermentation installation. Biowaste such as farmyard manure and organic material is fermented in this fermentation installation into a biogas mixture comprising CH_{4/}CO₂ and the remaining digestate. The digestate can be used as fertilizer in agriculture. The biogas mixture can be converted to heat and electricity by means of a combustion engine. Another option is to increase the energy content of this gas mixture by increasing the CH₄ content in this CH₄/CO₂ mixture by separating CO₂ therefrom.

Washing CO₂ out of the biogas mixture by means of water is known. Here the biogas mixture is set into counter-flow relative to a water flow. Both flows have a high pressure (for instance 10 bar(a)), and CO₂ molecules from the gas mixture dissolve in the liquid as a result of this high pressure. In order to maximize the contact surface between the liquid and the gas phases use is made of a stacked column (tower), where the water flows downward from the top and the gas mixture upward from the bottom. The pressure of the CO₂-saturated water is reduced in a second step, whereby the dissolved CO₂ molecules are once again released in the gas phase. After compression of this water it can be used once again in the adsorption phase. Described is that a purity of 97% can be realized with a yield of 97% and higher.

Washing out CO₂ with a liquid has the drawback that it requires a compression step, wherein both the biogas and the washing liquid are brought to a determined pressure, or a thermal regeneration step wherein the CO₂-saturated washing liquid is regenerated. Both a compression step and a thermal regeneration step use a relatively large amount of energy, and are thereby relatively expensive.

EP 0 314 040 A1 discloses a method and a device for the separation of carbon dioxide and methane in a city gas production, which method comprises the steps of adsorption of the carbon dioxide gas and the moisture from the reformed gas produced, depressurization and repressurization. The adsorbent used is carbon molecular sieve. The pressure of the incoming gas mixture is 9 bar, whereas the exhaust gas is removed at sub-atmospheric pressures down to 0.07 bar.

Known from CN 102391898 A is a method for separating and purifying carbon dioxide in marsh gas by applying a *pressure swing adsorption* (PSA) process. According to this method, carbon dioxide molecules from the marsh gas are adsorbed under high pressure to an absorbent, which then releases the carbon dioxide modules at a low pressure. This method also has the drawback that the marsh gas has to be brought to pressure, making the method relatively expensive.

It is an object of the invention to provide a method according to which it is possible in simple and cost-saving manner to separate methane gas and carbon dioxide gas from biogas.

It is a further object to provide the separated methane at a purity of 91% to 99.9%, measured in volumetric concentrations (% by volume). At these concentrations the separated methane is upgraded to biomethane with a calorific value which meets the quality standards set for biomethane in most industrialized countries.

This object is achieved, and other advantages realized, with a method according to the preamble which is performed according to the invention in accordance with a *vacuum swing adsorption process (VSA process),* wherein admitting the first gas mixture during step (ii) takes place under an absolute pressure of a maximum of 1.5 bar and discharging the third gas mixture during step (iv) takes place under an absolute pressure of a maximum of 0.5 bar, and wherein the adsorption mass comprises *carbon molecular sieve* (CMS) material.

A method for separating carbon dioxide and methane according to the invention provides the advantage that, owing to the absence of steps comprising compression or thermal regeneration, it requires relatively little energy. It has moreover been found that, with an adsorption mass with CMS material, exceptionally high methane yields (for instance 90-99%) are realized with a purity of at least 97% by volume of methane.

In an embodiment of a method according to the invention, admitting the first gas mixture as according to step (ii) is performed using a fan or blower.

In another embodiment discharging the second gas mixture as according to step (iii) is performed using a fan or blower.

An exceptionally high methane yield is achieved when the third gas mixture discharged as according to step (iv) is added to a first gas mixture to be admitted as according to step (ii).

In another embodiment the residence time of the first gas mixture admitted into the vessel as according to step (ii), which is defined as the quotient of the empty volume of the vessel and the flow rate of the first gas mixture admitted as according to step (ii), has a value in the range between 6 sec. and 36 sec.

It has been found that the yield and the purity of methane to be separated from the first gas mixture are optimal at such a residence time.

In an advantageous embodiment the *carbon molecular sieve* (CMS) material comprises pores of a diameter in the range between 0.2 nm and 0.4 nm.

The invention also relates to a device for separating a first gas mixture comprising carbon dioxide (CO₂) and methane (CH₄) into a second gas mixture with a relatively high methane content and a third gas mixture with a relatively high carbon dioxide content, comprising a number of vessels, wherein each vessel has at least one inlet and one outlet, and an adsorption mass comprising *carbon molecular sieve* (CMS) *material* for absorbing carbon dioxide is provided in each of these vessels, wherein the inlet of each of these vessels is connected to an inlet conduit in which a blower or fan is provided and wherein the outlet of each of these vessels is connected to an outlet conduit in which a blower or fan is provided.

In an embodiment the inlet of each of these vessels is connected to an outlet conduit for the third gas mixture in which a blower or fan is provided.

The invention will be elucidated hereinbelow on the basis of an exemplary embodiment, with reference to the drawing.

In the drawing
Fig. 1 shows a schematic view of a device according to the invention for separating a first gas mixture, for instance biogas, comprising carbon dioxide (CO₂) and methane (CH₄), and
Fig. 2 shows a graph of the pressure as a function of time in an adsorption vessel of the device shown in fig. 1.

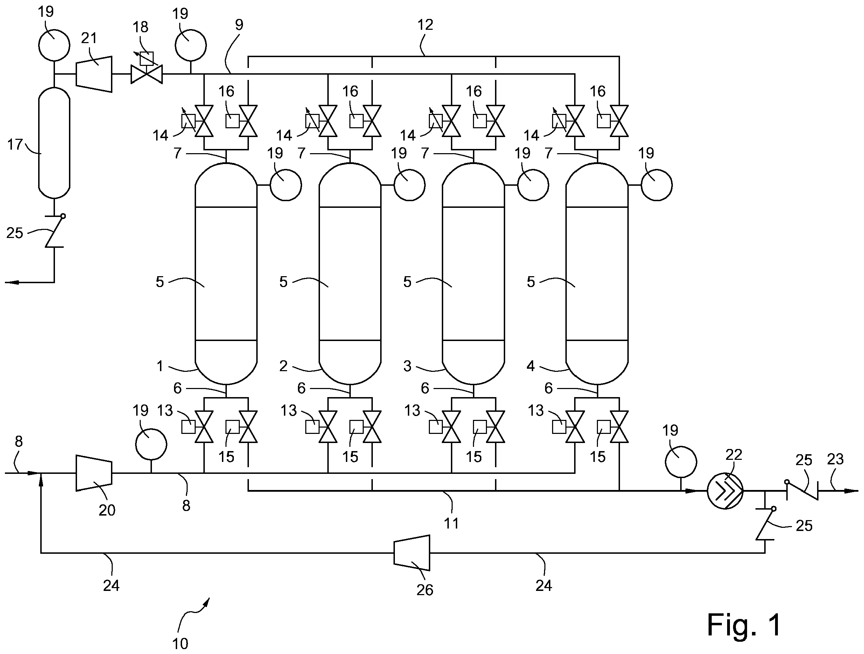

Fig. 1 shows a VSA device 10 comprising four vessels 1, 2, 3, 4 with pressure sensors 19, each having an adsorption bed, for instance an adsorption mass 5 with CMS material, and an inlet 6 and an outlet 7. Vessels 1, 2, 3, 4 are connected to four parallel conduits, i.e. a distribution conduit 8 with blower 20 for the gas mixture (biogas) for separating, a manifold 9 for the separated product gas (methane) with pressure sensor 19, valve 18 and blower 21, a manifold 11 for the separated waste gas (carbon dioxide) with non-return valve 25, pressure sensor 19 and vacuum pump 22, and a distribution conduit 12 for equalization and cleaning gas. Present in each of the conduits 8, 9, 11, 12 for each of the respective vessels 1, 2, 3, 4 is a feed valve 13, a product valve 14, a waste gas valve 15 and an equalization and cleaning valve 16. A product vessel 17 with non-return valve 18 and pressure sensor 19 are also shown. Carbon dioxide discharged with manifold 11 can be discharged via an outlet 23 to the outside world or can be fed back to distribution conduit 8 via a circulation conduit 24 with check valve 25 and blower 26.

### Example

The pressure of biogas with a pressure of 1 bar(a), which is supplied via distribution conduit 8, is increased to for instance 1.1 bar(a) using blower 20 and for instance guided into the first vessel for a determined period of time, the adsorption time. At the pressure of 1.1 bar (a) the carbon dioxide is adsorbed to the CMS material. The adsorption time is determined by the required end concentration of methane in the product gas. At a residence time, defined as the ratio of the (empty) bed volume (m³) and the normalized gas flow rate (m³/s at 1013 mbar and 0°C), of 27 sec. an optimal adsorption time was found to be 4 minutes. Following this adsorption process the pressure of the adsorption bed in vessel 1 is reduced by means of a first pressure-equalizing step with the adsorption bed in the second vessel 2. Following this step is a second pressure-equalizing step with the adsorption bed in third vessel 3. Depending on the number of available beds, this equalizing step can be repeated a number of times. After the equalizing steps, the bed can be further flushed clean with a gas mixture not comprising any carbon dioxide (purge gas). In this example the clean product gas is used for this purpose. The flow direction of this purge gas relative to the feed gas can be in both a co-flow and counterflow direction. The purge gas discharged from the vessel can be discharged or be admixed with the incoming biogas mixture (recirculation flow). Instead of using clean product gas as purge gas, use can also be made of nitrogen or air. After flushing of the bed the pressure is further reduced to 100 mbar(a) and lower using the vacuum pump. Each successive bed undergoes the same process as described for the first bed.

It has been found that, when the residence time is 27 sec. at a pressure of the supplied biogas of 1.1 bar(a), a purity of 99.1% by volume is achieved with a yield of 98.6% methane.

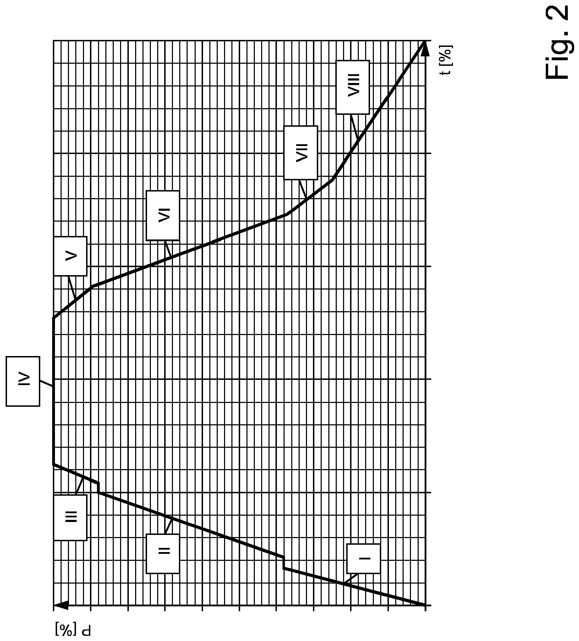

Fig. 2 shows the pressure in a vessel, in this case the first vessel 1 of VSA device 10, as percentage of the maximum pressure, as a function of time, as percentage of the time of a full operational cycle, for the successive steps of
(I) admitting separated product gas (methane) from second vessel 2 via outlet 7 of second vessel 2 and outlet 7 of first vessel 1 until the pressure in first vessel 1 has reached a predetermined value,
(II) admitting separated product gas from third vessel 3 via outlet 7 of third vessel 3 and outlet 7 of first vessel 1 until the pressure in first vessel 1 has reached a predetermined value,
(III) admitting separated product gas from fourth vessel 4 via outlet 7 of fourth vessel 4 and outlet 7 of first vessel 1 until the pressure in the first vessel has reached a predetermined value,
(IV) admitting biogas into first vessel 1 via inlet 6 for a predetermined period of time and simultaneously discharging separated product gas via outlet 7 of first vessel 1 to the product gas vessel 17,
(V) discharging separated product gas from first vessel 1 into third vessel 3 via outlets 7 of these vessels 1, 3,
(VI) discharging separated product gas from first vessel 1 into fourth vessel 4 via outlets 7 of these vessels 1, 4,
(VII) admitting purge gas, and
(VIII) discharging carbon dioxide-rich gas.

## Claims

1. Method for separating a first gas mixture comprising carbon dioxide (CO₂) and methane (CH₄) into a second gas mixture with a relatively high methane content and a third gas mixture with a relatively high carbon dioxide content, comprising the steps of
(i) providing the first gas mixture,
(ii) admitting the first gas mixture into at least one vessel (1, 2, 3, 4) in which an adsorption mass (5) for adsorbing carbon dioxide is received,
(iii) discharging the separated second gas mixture from the vessel (1, 2, 3, 4), and
(iv) discharging the third gas mixture from the vessel (1, 2, 3, 4), **characterized in that**
the method is performed in accordance with a *vacuum swing adsorption process (VSA process),* wherein
admitting the first gas mixture during step (ii) takes place under an absolute pressure of a maximum of 1.5 bar, and
discharging the third gas mixture during step (iv) takes place under an absolute pressure of a maximum of 0.5 bar, and
wherein the adsorption mass (5) comprises *carbon molecular sieve* (CMS) material.

2. Method as claimed in claim 1, wherein admitting the first gas mixture as according to step (ii) is performed using a fan or blower (20).

3. Method as claimed in any of the claims 1-2, wherein discharging the second gas mixture as according to step (iii) is performed using a fan or blower (21).

4. Method as claimed in any of the claims 1-3, wherein the third gas mixture discharged as according to step (iv) is added to a first gas mixture to be admitted as according to step (ii).

5. Method as claimed in any of the claims 1-4, wherein the quotient of the empty volume of the vessel (1, 2, 3, 4) and the flow rate of the first gas mixture admitted as according to step (ii) has a value in the range between 6 sec. and 36 sec.

6. Method as claimed in any of the claims 1-5, wherein the *carbon molecular sieve* (CMS) material comprises pores of a diameter in the range between 0.2 nm and 0.4 nm.

7. Method as claimed in any of the claims 1-6, wherein the first gas mixture is admitted as according to step (ii) for a period of 2 to 8 minutes.

8. Device (10) for separating a first gas mixture comprising carbon dioxide (CO₂) and methane (CH₄) into a second gas mixture with a relatively high methane content and a third gas mixture with a relatively high carbon dioxide content, comprising a number of vessels (1, 2, 3, 4), wherein each vessel (1, 2, 3, 4) has at least one inlet (6) and one outlet (7), and an adsorption mass (5) comprising *carbon molecular sieve* (CMS) material for absorbing carbon dioxide is provided in each of these vessels (1, 2, 3, 4), **characterized in that** the inlet (6) of each of these vessels (1, 2, 3, 4) is connected to an inlet conduit (8) in which a blower or fan (20) is provided, and the outlet (7) of each of these vessels (1, 2, 3, 4) is connected to an outlet conduit (9) in which a blower or fan (21) is provided.

9. Device as claimed in claim 8, **characterized in that** the inlet (6) of each of these vessels (1, 2, 3, 4) is connected to an outlet conduit (11) for the third gas mixture in which a vacuum pump (22) is provided.

10. Device as claimed in claim 8, **characterized in that** the inlet (6) of each of these vessels (1, 2, 3, 4) is connected to an outlet conduit (11) for the third gas mixture in which a fan or blower is provided.

## Patentansprüche

1. Verfahren zur Trennung eines Kohlendioxids (CO₂) und Methan (CH₄) enthaltenden erstes Gasgemisch in ein zweites Gasgemisch mit einem relativ hohen Methangehalt und in ein drittes Gasgemisch mit einem relativ hohen Kohlendioxidgehalt, umfassend die Schritte
(i) des Bereitstellens des ersten Gasgemisches,
(ii) des Einlassens des ersten Gasgemisches in zumindest einen Behälter (1, 2, 3, 4), in dem eine Adsorptionsmasse (5) zur Adsorbierung von Kohlendioxid aufgenommen ist,
(iii) des Auslassens des abgetrennten zweiten Gasgemisches aus dem Behälter (1, 2, 3, 4), und
(iv) des Auslassens des dritten Gasgemisches aus dem Behälter (1, 2, 3, 4), **dadurch gekennzeichnet,**
**dass** das Verfahren gemäß einer Vakuumwechseladsorption *(VSA Verfahren)* ausgeführt wird, wobei
das während des Schrittes (ii) Einlassen des ersten Gasgemisches unter einem absoluten Druck von höchstens 1,5 bar erfolgt, und
das während des Schrittes (iv) Auslassen des dritten Gasgemisches unter einem absoluten Druck von höchstens 0,5 bar erfolgt, und
wobei die Adsorptionsmasse (5) Kohlenstoff-Molekularsieb (CMS) Material umfasst.

2. Verfahren gemäß Anspruch 1, wobei das gemäß dem Schritt (ii) Einlassen des ersten Gasgemisches mittels eines Ventilators oder Gebläses (20) ausgeführt wird.

3. Verfahren gemäß einem der Ansprüche 1-2, wobei das gemäß dem Schritt (iii) Auslassen des zweiten Gasgemisches mittels eines Ventilators oder Gebläses (21) ausgeführt wird.

4. Verfahren gemäß einem der Ansprüche 1-3, wobei das gemäß dem Schritt (iv) ausgelassene dritte Gasgemisch einem gemäß dem Schritt (ii) einzulassenden ersten Gasgemisch zugefügt wird.

5. Verfahren gemäß einem der Ansprüche 1-4, wobei der Quotient des leeren Volumens des Behälters (1, 2, 3, 4) und der Fließgeschwindigkeit des gemäß dem Schritt (ii) eingelassenen ersten Gasgemisches einen Wert in dem Bereich zwischen 6 Sek. und 36 Sek. hat.

6. Verfahren gemäß einem der Ansprüche 1-5, wobei das Kohlenstoff-Molekularsieb (CMS) Material Poren mit einem Durchmesser in dem Bereich zwischen 0,2 nm und 0,4 nm enthält.

7. Verfahren gemäß einem der Ansprüche 1-6, wobei das erste Gasgemisch gemäß dem Schritt (ii) während eines Zeitraumes von 2 bis 8 Minuten eingelassen wird.

8. Einrichtung (10) zur Trennung eines Kohlendioxid (CO₂) und Methan (CH₄) enthaltenden ersten Gasgemisches in ein zweites Gasgemisch mit einem relativ hohen Methangehalt und in ein drittes Gasgemisch mit einem relativ hohen Kohlendioxidgehalt, umfassend eine Mehrzahl von Behältern (1, 2, 3, 4), wobei jeder Behälter (1, 2, 3, 4) mindestens einen Einlass (6) und einen Auslass (7) hat, und in jedem dieser Behälter (1, 2, 3, 4) eine Adsorptionsmasse (5), die Kohlenstoff-Molekularsieb (CMS) Material umfasst zur Absorbierung von Kohlendioxid bereitgestellt ist, **dadurch gekennzeichnet, dass** der Einlass (6) von jedem dieser Behälter (1, 2, 3, 4) mit einer Einlassleitung (8) verbunden ist, in der ein Gebläse oder Ventilator (20) bereitgestellt ist, und der Auslass (7) von jedem dieser Behälter (1, 2, 3, 4) mit einer Auslassleitung (9) verbunden ist, in der ein Gebläse oder Ventilator (21) bereitgestellt ist.

9. Einrichtung gemäß dem Anspruch 8, **dadurch gekennzeichnet, dass** der Einlass (6) von jedem dieser Behälter (1, 2, 3, 4) mit einer Auslassleitung (11) für das dritte Gasgemisch verbunden ist, in der eine Vakuumpumpe (22) bereitgestellt ist.

10. Einrichtung gemäß dem Anspruch 8, **dadurch gekennzeichnet, dass** der Einlass (6) von jedem dieser Behälter (1, 2, 3, 4) mit einer Auslassleitung (11) für das dritte Gasgemisch verbunden ist, in der ein Ventilator oder Gebläse bereitgestellt ist.

## Revendications

1. Procédé pour séparer un premier mélange de gaz contenant du dioxyde de carbone (CO₂) et du méthane (CH₄) en un deuxième mélange de gaz présentant une teneur en méthane relativement élevée et un troisième mélange de gaz présentant une teneur en dioxyde de carbone relativement élevée, comprenant les étapes suivantes:
(i) fournir le premier mélange de gaz,
(ii) admettre le premier mélange de gaz dans au moins une cuve (1, 2, 3, 4) dans laquelle une masse d'adsorption (5) pour adsorber du dioxyde de carbone est reçue,
(iii) décharger le deuxième mélange de gaz séparé de la cuve (1, 2, 3, 4), et
(iv) décharger le troisième mélange de gaz de la cuve (1, 2, 3, 4),
**caractérisé en ce que** le procédé est exécuté suivant un procédé d'adsorption à oscillation sous vide (procédé VSA), dans lequel
l'admission du premier mélange de gaz pendant l'étape (ii) se produit sous une pression absolue d'un maximum de 1,5 bars, et
la décharge du troisième mélange de gaz pendant l'étape (iv) se produit sous une pression absolue d'un maximum de 0,5 bar, et
dans lequel la masse d'adsorption (5) comprend un matériau de tamis moléculaire au carbone (CMS).

2. Procédé selon la revendication 1, dans lequel l'admission du premier mélange de gaz selon l'étape (ii) est exécutée en utilisant un ventilateur ou une soufflante (20) .

3. Procédé selon l'une quelconque des revendications 1 à 2, dans lequel la décharge du deuxième mélange de gaz selon l'étape (iii) est exécutée en utilisant un ventilateur ou une soufflante (21) .

4. Procédé selon l'une quelconque des revendications 1 à 3, dans lequel le troisième mélange de gaz déchargé selon l'étape (iv) est ajouté à un premier mélange de gaz à admettre selon l'étape (ii).

5. Procédé selon l'une quelconque des revendications 1 à 4, dans lequel le quotient du volume vide de la cuve (1, 2, 3, 4) et le débit du premier mélange de gaz admis selon l'étape (ii) présente une valeur comprise dans la gamme entre 6 secondes et 36 secondes.

6. Procédé selon l'une quelconque des revendications 1 à 5, dans lequel le matériau de tamis moléculaire au carbone (CMS) comprend des pores d'un diamètre compris dans la gamme entre 0,2 nm et 0,4 nm.

7. Procédé selon l'une quelconque des revendications 1 à 6, dans lequel le premier mélange de gaz est admis selon l'étape (ii) pendant une période de 2 à 8 minutes.

8. Dispositif (10) pour séparer un premier mélange de gaz contenant du dioxyde de carbone (CO₂) et du méthane (CH₄) en un deuxième mélange de gaz présentant une teneur en méthane relativement élevée et un troisième mélange de gaz présentant une teneur en dioxyde de carbone relativement élevée, comprenant un certain nombre de cuves (1, 2, 3, 4), dans lequel chaque cuve (1, 2, 3, 4) comporte au moins une entrée (6) et une sortie (7), et une masse d'adsorption (5) comprenant un matériau de tamis moléculaire au carbone (CMS) pour absorber du dioxyde de carbone est prévue dans chacune de ces cuves (1, 2, 3, 4),
**caractérisé en ce que** l'entrée (6) de chacune de ces cuves (1, 2, 3, 4) est connectée à un conduit d'entrée (8) dans lequel il est prévu une soufflante ou un ventilateur (20), et la sortie (7) de chacune de ces cuves (1, 2, 3, 4) est connectée à un conduit de sortie (9) dans lequel il est prévu une soufflante ou un ventilateur (21).

9. Dispositif selon la revendication 8,
**caractérisé en ce que** l'entrée (6) de chacune de ces cuves (1, 2, 3, 4) est connectée à un conduit de sortie (11) pour le troisième mélange de gaz dans lequel il est prévu une pompe à vide (22).

10. Dispositif selon la revendication 8,
**caractérisé en ce que** l'entrée (6) de chacune de ces cuves (1, 2, 3, 4) est connectée à un conduit de sortie (11) pour le troisième mélange de gaz dans lequel il est prévu un ventilateur ou une soufflante.
